(19) Europäisches Patentamt
European Patent Office
Office européen des brevets

(11) Veröffentlichungsnummer: **0 091 056**
**A2**

(12) # EUROPÄISCHE PATENTANMELDUNG

(21) Anmeldenummer: 83103053.1

(22) Anmeldetag: 28.03.83

(51) Int. Cl.³: C 07 D 233/61
C 07 D 233/60, C 07 D 233/56

(30) Priorität: 06.04.82 DE 3212749

(43) Veröffentlichungstag der Anmeldung: 12.10.83 Patentblatt 83/41

(84) Benannte Vertragsstaaten: BE CH DE FR GB LI

(71) Anmelder: BASF Aktiengesellschaft
Carl-Bosch-Strasse 38
D-6700 Ludwigshafen(DE)

(72) Erfinder: Dockner, Toni, Dr.
Grossgasse 6
D-6701 Meckenheim(DE)

(72) Erfinder: Frank, Anton
Bannwasserstrasse 72
D-6700 Ludwigshafen(DE)

(72) Erfinder: Kempe, Uwe, Dr.
Danziger Strasse 9
D-6701 Dannstadt-Schauernheim(DE)

(72) Erfinder: Wetzler, Matthias
Saarstrasse 8
D-6703 Limburgerhof(DE)

(72) Erfinder: Karn, Helmut
Sternstrasse 120
D-6700 Ludwigshafen(DE)

(54) **Neue 5-substituierte 4-Methylimidazole und Verfahren zu ihrer Herstellung.**

(57) Neue 5-substituierte 4-Methylimidazole und ein Verfahren zur Herstellung von 5-substituierten 4-Methylimidazolen durch Oxidation von 4-Methyl-5-hydroxymethylimidazolen und gewünschtenfalls Umsetzung der so gebildeten 4-Methyl-5-formylimidazole mit Anilinen, Hydroxylamin, Hydrazin, Semicarbazid, Thiosemicarbazid, Triphenylphosphincarbalkoxymethylenen oder Phosphonoessigsäurealkylestern und gegebenenfalls Hydrolyse der so gebildeten Ester.

Die nach dem Verfahren der Erfindung herstellbaren Endstoffe I sind wertvolle Ausgangsstoffe für Pflanzenschutzmittel, Farbstoffe und Pharmazeutika.

EP 0 091 056 A2

Croydon Printing Company Ltd.

Neue 5-substituierte 4-Methylimidazole und Verfahren zu ihrer Herstellung

Die Erfindung betrifft neue 5-substituierte 4-Methylimidazole und ein Verfahren zur Herstellung von 5-substituierten 4-Methylimidazolen durch Oxidation von 4-Methyl-5-hydroxymethylimidazolen und gewünschtenfalls Umsetzung der so gebildeten 4-Methyl-5-formylimidazole mit Anilinen, Hydroxylamin, Hydrazin, Semicarbazid, Thiosemicarbazid, Triphenylphosphin-carbalkoxymethylenen oder Phosphonoessigsäurealkylestern und gegebenenfalls Hydrolyse der so gebildeten Ester.

Es ist aus Houben-Weyl, Methoden der Organischen Chemie, Band 7/1, Seiten 180 bis 183 bekannt, primäre Alkoholgruppen mit Salpetersäure zu Aldehyden umzusetzen. Temperaturen von 30°C und 45-gewichtsprozentige bis 100-gewichtsprozentige Salpetersäure werden, gegebenenfalls zusammen mit 70-gewichtsprozentiger Schwefelsäure, als Merkmale des Verfahrens angeführt. Es wird darauf hingewiesen, daß die Aldehydgruppe gegen konzentrierte Salpetersäure nur bei niederen Temperaturen relativ stabil ist.

Rodd, Chemistry of Carbon Compounds (1. Auflage, Elsevier, N.Y.), Band IV A, Seite 298, gibt für die Herstellung von Imidazol-4-aldehyd und 2-Phenyl-5-methylimidazol-4-aldehyd ebenfalls die Oxidation des 4-Hydroxymethylderivats der beiden Imidazole mit Salpetersäure an. Auch 5-Methylimidazol-4-aldehyd wird erwähnt.

1-Methylimidazol-5-aldehyd kann durch Umsetzung eines entsprechenden Carbonsäureesters mit Hydrazinhydrat, Kondensation des gebildeten Hydrazids mit Benzolsulfochlorid und Zersetzung des gebildeten Acylbenzolsulfonyldihydrazids in Glykol mit Soda bei 160°C zum Aldehyd hergestellt

WB/P

werden.

Es wurde nun gefunden, daß man 5-substituierte 4-Methylimidazole der Formel

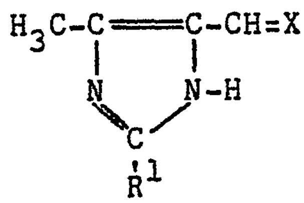

I,

worin X den Rest 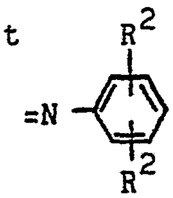 , den Rest =NOH, den Rest $=N-NH_2$, den Rest 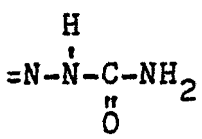, den Rest 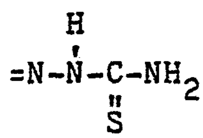 oder den Rest $=CH-COOR^3$ bedeutet, $R^1$, $R^2$ und $R^3$ gleich oder verschieden sein können und jeweils ein Wasserstoffatom oder einen aliphatischen Rest bezeichnen und $R^2$ auch für ein Halogenatom oder eine Alkoxygruppe steht, X auch, wenn $R^1$ einen aliphatischen Rest bedeutet, ein Sauerstoffatom bezeichnen kann, vorteilhaft erhält, wenn man 4-Methyl-5-hydroxymethylimidazole der Formel

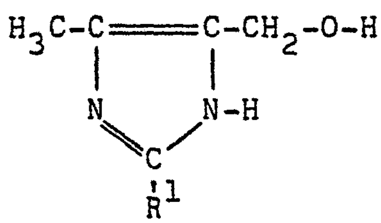

II,

worin $R^1$ die vorgenannte Bedeutung besitzt, mit Oxidationsmitteln zu 4-Methyl-5-formylimidazolen der Formel

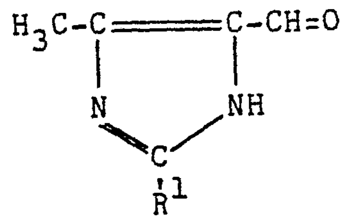 III,

worin $R^1$ die vorgenannte Bedeutung besitzt, oxidiert und gewünschtenfalls die Stoffe III in üblicher Weise mit Stoffen der Formel

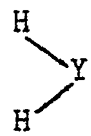 IV,

worin $R^2$ und $R^3$ die vorgenannte Bedeutung besitzen, und Y den Rest

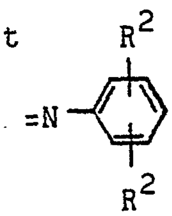 , den Rest =NOH, den Rest $=N-NH_2$,

den Rest 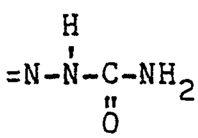 oder den Rest 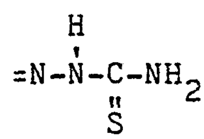 bezeichnet, oder mit Triphenylphosphin-carbalkoxymethylenen oder Dialkoxyphosphonoessigsäurealkylestern umsetzt und gewünschtenfalls die so erhaltenen ß-(4-Methyl)-imidazolyl-(5)-acrylsäurealkylester der Formel

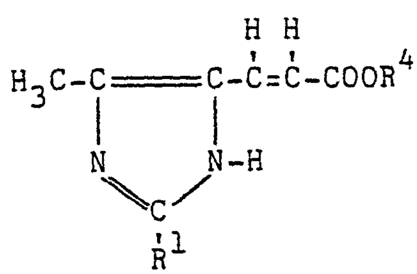 V,

worin $R^1$ die vorgenannte Bedeutung besitzt und $R^4$ einen aliphatischen Rest bezeichnet, in üblicher Weise zu Acrylsäuren der Formel

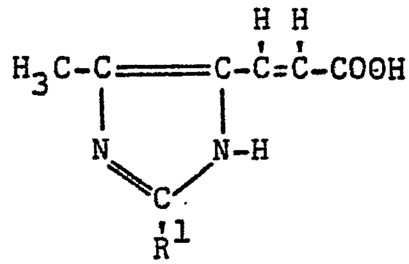 VI,

worin $R^1$ die vorgenannte Bedeutung besitzt, hydrolysiert.

Weiterhin wurden die neuen 5-substituierten 4-Methylimidazole der Formel

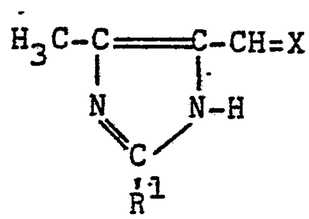 I,

worin X den Rest

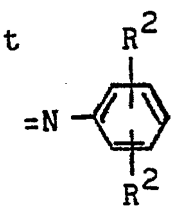 , den Rest =NOH, den Rest $=N-NH_2$,

den Rest 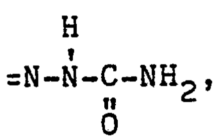, den Rest 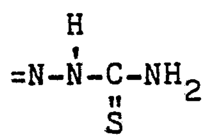 oder den Rest $=CH-COOR^3$ bedeutet, $R^1$, $R^2$ und $R^3$ gleich oder verschieden sein können und jeweils ein Wasserstoffatom oder einen aliphatischen Rest bezeichnen und $R^2$ auch für ein Halogenatom oder eine Alkoxygruppe steht, X auch, wenn $R^1$ einen aliphatischen Rest bedeutet, ein Sauerstoffatom bezeichnen kann, gefunden.

Die Umsetzung kann für den Fall der Verwendung von 4-Methyl-5-hydroxymethylimidazol, Salpetersäure, Hydroxylamin, Triphenylphosphin-carbmethoxymethylen und p-Diethoxyphosphonoessigsäureethylester durch die folgenden Formeln wiedergegeben werden:

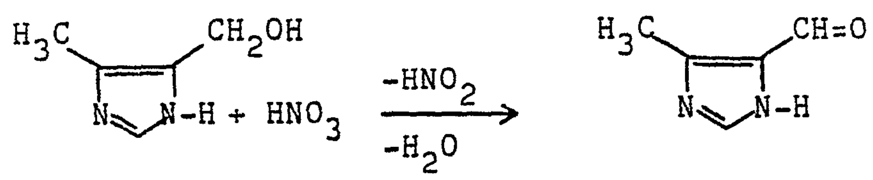


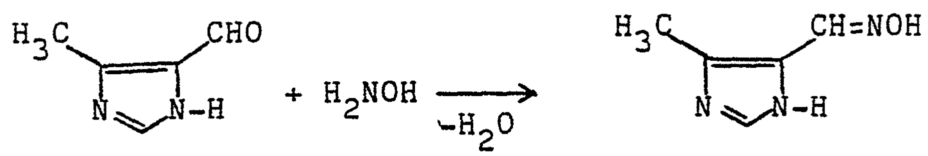


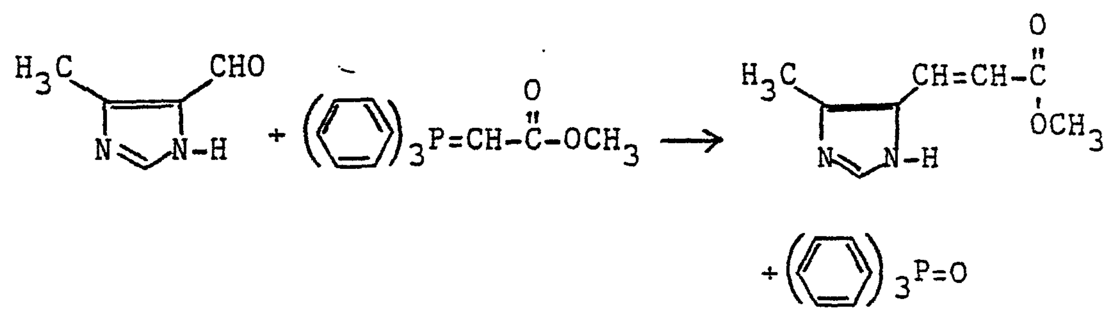

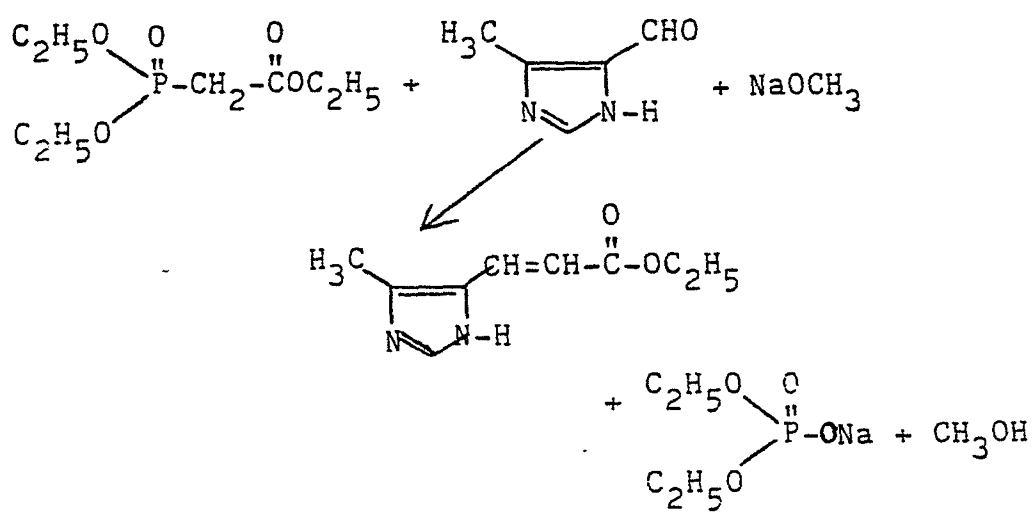

Im Hinblick auf die bekannten Verfahren liefert das Verfahren nach der Erfindung auf einfachem und wirtschaftlichem Wege 4-Methyl-5-formylimidazole sowie die neuen 5-substituierten 4-Methylimidazole in guter Ausbeute und Reinheit. Die neuen Stoffe sind überraschend als spezielle Herbizide und Hautschutzmittel verwendbar.

Die 4-Methyl-5-hydroxymethylimidazole II werden in üblicher Weise, z.B. (J. Chem. Soc., Band 99 (1911), Seiten 2052 bis 2059) durch Umsetzung von 4-Methylglyoxalin mit 40-prozentiger Formaldehydlösung bei 120°C, hergestellt.

Als Triphenylphosphin-carbalkoxymethylene kommen solche der Formel

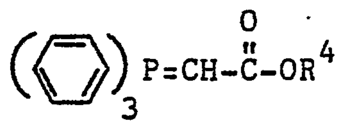 VII,

als Dialkoxyphosphonoessigsäurealkylester solche der Formel

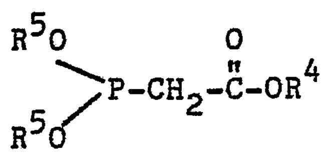 VIII,

worin $R^4$ und die einzelnen Reste $R^5$ gleich oder verschieden sein können und jeweils einen aliphatischen Rest bezeichnen, in Betracht.

Bevorzugte Ausgangsstoffe II, IV, VII und VIII und dementsprechend bevorzugte Stoffe III und Endstoffe I sind solche, in deren Formeln $R^1$, $R^2$ und $R^3$ gleich oder verschieden sein können und jeweils ein Wasserstoffatom oder einen Alkylrest mit 1 bis 18 Kohlenstoffatomen, bevorzugt 1 bis 10, insbesondere mit 1 bis 4 Kohlenstoffatomen, bezeichnen, X den Rest

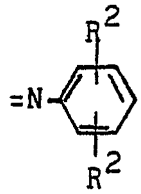 , den Rest =NOH, den Rest $=N-NH_2$, den Rest

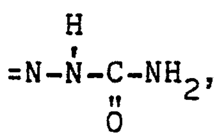, den Rest 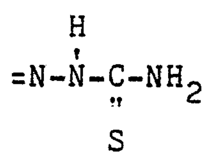 oder den Rest $=CH-COOR^3$

bedeutet, $R^2$ auch ein Bromatom oder insbesondere ein Chloratom oder eine Alkoxygruppe mit 1 bis 4 Kohlenstoffatomen bezeichnet, Y den Rest

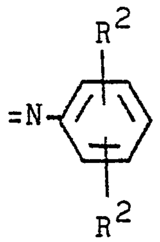

, den Rest =NOH, den Rest $=N-NH_2$, den Rest $=N-\overset{H}{N}-\underset{O}{\overset{\|}{C}}-NH_2$

oder den Rest $=N-\overset{H}{N}-\underset{S}{\overset{\|}{C}}-NH_2$ bezeichnet, X auch, wenn $R^1$ einen aliphatischen Rest bedeutet, ein Sauerstoffatom bezeichnen kann, $R^4$ und die

einzelnen Reste $R^5$ gleich oder verschieden sein können und jeweils einen Alkylrest mit 1 bis 18 Kohlenstoffatomen, bevorzugt 1 bis 10, insbesondere mit 1 bis 4 Kohlenstoffatomen, bezeichnen. Die vorgenannten Reste können noch durch unter den Reaktionsbedingungen inerte Gruppen, z.B. Alkylgruppen oder Alkoxygruppen mit jeweils 1 bis 4 Kohlenstoffatomen, substituiert sein.

Als Ausgangsstoffe II kommen z.B. in Frage: In 2-Stellung durch die Methyl-, Ethyl-, Propyl-, Isopropyl-, Butyl-, Isobutyl-, Pentyl-, sek.-Butyl-, tert.-Butyl-, Hexyl-, Heptyl-, Octyl-, Nonyl-, Decyl-, Octadecyl-gruppe substituierte 4-Methyl-5-hydroxymethylimidazole; unsubstituiertes 4-Methyl-5-hydroxymethylimidazol.

Als Ausgangsstoffe IV sind z.B. geeignet: Anilin; in 2-, 3- oder 4-Stellung einfach oder in 2,3-, 2,4-, 2,5-, 2,6-, 3,4-, 3,5-Stellung gleich oder unterschiedlich zweifach durch Chlor, Brom, Methyl-, Ethyl-, Propyl-, Isopropyl-, Butyl-, Isobutyl-, Pentyl-, sek.-Butyl-, tert.-Butyl-, Methoxy-, Ethoxy-, Propoxy-, Isopropoxy-, Butoxy-, Isobutoxy-, sek.-Butoxy-, tert.-Butoxy-gruppen substituierte Aniline; Hydroxylamin, Hydrazin, Semicarbazid, Thiosemicarbazid.

Als weitere Ausgangsstoffe sind z.B. Triphenylphosphincarbalkoxymethylene oder Dialkoxyphosphonoessigsäurealkylester geeignet, deren Ester- bzw. Alkoxygruppe z.B. eine Methoxy-, Ethoxy-, Propoxy-, Isopropoxy-, Butoxy-, Isobutoxy-, sek.-Butoxy-, tert.-Butoxy-, Pentyloxy-, Hexyloxy-, Heptyloxy-, Octyloxy-, Nonyloxy-, Decyloxy-gruppe ist. Die aus diesen Estern gebildete ß-(4-Methyl)-imidazolyl-(5)-acrylsäurealkylester sind z.B. auch für eine nachfolgende Hydrolyse zu entsprechenden Acrylsäuren vorteilhaft.

Als Oxidationsmittel wird zweckmäßig Salpetersäure, vorteilhaft in einem Molverhältnis von 3 bis 4 zu 1 Ausgangsstoff II und in Gestalt von 30 bis 80, insbesondere von 40 bis 70, bevorzugt von 63 bis 65-gewichtsprozentigen wäßrigen Lösungen, verwendet. Die Umsetzung wird im allgemeinen bei einer Temperatur von 30 bis 100, bevorzugt 40 bis 90°C, drucklos oder unter Druck, kontinuierlich oder diskontinuierlich durchgeführt. Als zusätzliches Lösungsmittel wird bevorzugt Wasser, zweckmäßig in einer Menge (zusätzlich zu der in der verdünnten Salpetersäure vorhandenen Wassermenge) von 100 bis 120 Gewichtsprozent, bezogen auf Ausgangsstoff II, verwendet. Bezüglich der Reaktionsführung wird auch auf das vorgenannte Werk von Houben-Weyl (loc. cit) verwiesen. Vorteilhaft wird Ausgangsstoff II in Gestalt seines Hydrochlorids verwendet.

Aus den so gebildeten 4-Methyl-5-formylimidazolen III können mit den Ausgangsstoffen IV, Triphenylphosphincarbalkoxymethylenen oder Dialkoxy-phosphonoessigsäurealkylestern die entsprechenden Anile, Oxime, Hydrazone, Semicarbazone, Thiosemicarbazone oder Acrylsäureester I hergestellt werden.

Zur Herstellung der Anile werden die Ausgangsstoffe III und Aniline IV, zweckmäßig in einem Molverhältnis von 1 bis 11. Ausgangsstoff IV je Mol Ausgangsstoff III, vorteilhaft in Gegenwart von Säure, z.B. Salzsäure, bevorzugt in einer Menge von 0,1 bis 0,5 Äquivalenten Säure je Mol Ausgangsstoff III, umgesetzt. Die Reaktion wird im allgemeinen bei einer Temperatur von 80 bis 120 , bevorzugt 100 bis 105°C, drucklos oder unter Druck, kontinuierlich oder diskontinuierlich durchgeführt. Als zusätzliches Lösungsmittel wird bevorzugt Wasser, zweckmäßig in einer Menge (zusätzlich zu der in der Säure vorhandenen Wassermenge) von 500 bis 600 Gewichtsprozent, bezogen auf Ausgangsstoff III, verwendet. Bezüglich der Reaktionsführung wird auch auf das vorgenannte Werk von Houben-Weyl (loc. cit., Seiten 453 - 456)

verwiesen.

Zur Herstellung der Oxime und Hydrazone werden Ausgangsstoff III und Hydroxylamin bzw. Hydrazin, zweckmäßig in einem Molverhältnis von 1 bis 1,1 Hydroxylamin bzw. Hydrazin je Mol Ausgangsstoff III umgesetzt. Die Reaktion wird im allgemeinen bei einer Temperatur von 60 bis 120, bevorzugt 70 bis 100°C, drucklos oder unter Druck, kontinuierlich oder diskontinuierlich durchgeführt. Zweckmäßig werden organische Lösungsmittel, z.B. Alkohole wie Methanol, Ethanol, Propanol, Isopropanol, Butanol, Isobutanol, sek.-Butanol, tert.-Butanol, vorteilhaft in einer Menge von 100 bis 10 000 Gewichtsprozent, bezogen auf Ausgangsstoff III, verwendet. Bezüglich der Reaktionsführung wird auf Houben-Weyl (loc. cit., Seiten 461 - 468 und 471 - 474) verwiesen. Zweckmäßig wird Hydroxylamin in Gestalt seiner Salze, z.B. des Chlorids, verwendet. Ebenfalls kommt zweckmäßig Hydrazin in Gestalt seines Salzes, z.B. des Chlorids, oder bevorzugt in Gestalt seines Hydrats in Frage.

Zur Herstellung der Semicarbazone und Thiosemicarbazone werden Ausgangsstoff III und Semicarbazid bzw. Thiosemicarbazid, zweckmäßig in einem Molverhältnis von 1 bis 1,1 Semicarbazid bzw. Thiosemicarbazid je Mol Ausgangstoff III umgesetzt. Die Reaktion wird im Allgemeinen bei einer Temperatur von 60 bis 120, bevorzugt 70 bis 110°C, drucklos oder unter Druck, kontinuierlich oder diskontinuierlich durchgeführt. Als zusätzliches Lösungsmittel werden zweckmäßig Alkohole und bevorzugt Alkohol/Wasser-Gemische, vorteilhaft in einem Verhältnis von 1 bis 5 Mol Alkohol je Mol Wasser und in einer Menge von 10 bis 50 Mol Alkohol je Mol Ausgangsstoff III verwendet. Als Alkohole kommen bevorzugt Methanol, Ethanol, Propanol, Isopropanol, Butanol, Isobutanol, sek.-Butanol, tert.-Butanol, in Betracht. Bezüglich der Reaktionsführung wird auf Houben-Weyl (loc. cit., Seiten 468 - 471) verwiesen. Die Ausgangsstoffe IV können in Gestalt der freien Base, vorteilhaft im

Falle von Thiosemicarbazid, oder ihrer Salze, z.B. Hydrochlorid, vorteilhaft im Falle von Semicarbazid, verwendet werden.

Zur Herstellung der Acrylsäureester werden Ausgangsstoff III und Triphenylphosphin-carbalkoxymethylene oder Dialkoxy-phosphonoessigsäurealkylester, zweckmäßig in einem Molverhältnis von 1 bis 1,1 Mol Triphenylphosphin-carbalkoxymethylene oder 1 bis 1,1 Mol Phosphonessigsäurealkylester je Mol Ausgangsstoff III, umgesetzt. Die Umsetzung wird im allgemeinen bei einer Temperatur von 50 bis 100, bevorzugt 50 bis 90°C, drucklos oder unter Druck, kontinuierlich oder diskontinuierlich durchgeführt. Zweckmäßig werden organische Lösungsmittel, z.B. Alkohole wie Methanol, Ethanol, Propanol, Isopropanol, Butanol, Isobutanol, sek.-Butanol, tert.-Butanol, vorteilhaft in einer Menge von 100 bis 10 000 Gewichtsprozent, bezogen auf Ausgangsstoff III, verwendet. Im Falle der Verwendung von Phosphonoessigsäurealkylestern wird zweckmäßig noch ein Alkoholat, vorteilhaft mit 1 bis 4 Kohlenstoffatomen und Natrium oder Kalium als Kation, z.B. Natriummethylat, in Gestalt seiner Lösung, zweckmäßig in dem entsprechenden Alkohol, z.B. Methanol, vorzugsweise in einer Menge von 1 bis 1,1 Mol Alkoholat je Mol Ausgangsstoff III, zugesetzt.

Zur Herstellung der 4-Methylimidazolyl-5-acrylsäure werden vorgenannte Ester in üblicher Weise verseift, zweckmäßig in salzsaurem Medium, vorteilhaft in einer Menge von 4 bis 8 Mol Salzsäure je Mol Ester. Die Verseifung wird im allgemeinen bei einer Temperatur von 60 bis 140, bevorzugt 70 bis 105°C, drucklos oder unter Druck, kontinuierlich oder diskontinuierlich durchgeführt. Als zusätzliches Lösungsmittel wird bevorzugt Wasser, zweckmäßig in einer Menge (zusätzlich zur in der wäßrigen Salzsäure vorhandenen Wassermenge) von 200 bis 300 Gewichtsprozent, bezogen auf zu verseifenden Ester, verwendet.

Der Ester kommt zweckmäßig in Gestalt seiner Salze, z.B. des Hydrochlorids, zur Umsetzung.

Die allgemeine Reaktion wird wie folgt durchgeführt: Ein Gemisch der vorgenannten Ausgangsstoffe, Lösungsmittel und gegebenenfalls Säure wird während 0,2 bis 7 Stunden bei der angegebenen Reaktionstemperatur umgesetzt. Aus dem Reaktionsgemisch wird der Endstoff in üblicher Weise isoliert. Beispielsweise wird ein Stoff III durch Abkühlen, Einstellen eines pH von 8,5 und Filtration; ein Anil I durch Abkühlen und Filtration; ein Oxim, Hydrazon oder Thiosemicarbazon I durch Eindampfen des Reaktionsgemisches und Umkristallisation des Rückstandes; ein Semicarbazon durch Abkühlen, Filtration, Behandlung mit Base, z.B. Ammoniakwasser, und Filtration; ein Acrylsäureester I durch Abkühlen, Ansäuern und Filtration; eine Acrylsäure I durch Abkühlen, Filtration, Lösen des Filtergutes in Wasser, Einstellen eines pH von 5,4, Filtration; abgetrennt.

Die nach dem Verfahren der Erfindung herstellbaren Endstoffe I sind wertvolle Ausgangsstoffe für Pflanzenschutzmittel, Farbstoffe und Pharmazeutika. Zum Beispiel ist der Cu-Komplex des Thiosemicarbazons

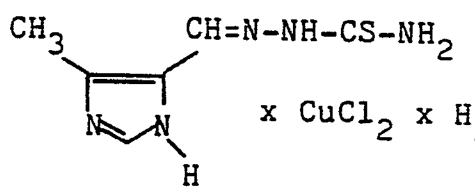

x $CuCl_2$ x $H_2O$

fungizid und wirkt sehr gut gegen Septoria. Das freie Thiosemicarbazon wirkt gegen Weizenmehltau. Ebenfalls wirksam gegen Weizenmehltau ist das Anil

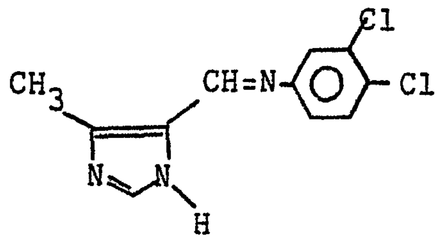

4-Methyl-imidazolyl-5-acrylsäure

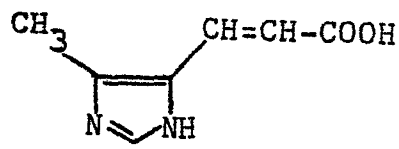

ist ein Hautschutzmittel. Durch die Verfügbarkeit des Imidazolaldehyds besteht nun die Möglichkeit eines synthetischen Zugangs zu den als Lichtschutzmittel eingesetzten Verbindungen, z.B. 4-Methyl-imidazol-5-acrylsäure.

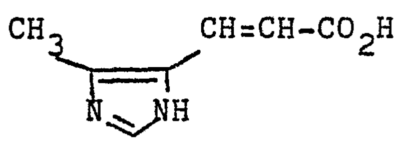

Beispiel 1

375 Gramm 4-Methyl-5-hydroxymethylimidazol-hydrochlorid wurden in einer Mischung von 800 Gramm Salpetersäure (65 Gew.%) und 400 Gramm Wasser gelöst und auf 50°C erwärmt. Bei dieser Temperatur setzte unter Entwicklung von Stickoxiden eine schwach exotherme Reaktion ein. Man hielt die Temperatur 2 Stunden bei 50 bis 55°C. Nach Abklingen der exothermen Reaktion rührte man 4 Stunden bei 80°C nach, kühlte das Gemisch im Eisbad ab und stellte das Reaktionsgemisch

durch Zugabe von 180 Milliliter Natronlauge (50 Gew.%) auf pH = 8,5. Man kühlte das Gemisch 4 Stunden im Eisbad auf 0 bis 5°C, saugte den Niederschlag ab, wusch das Gemisch mit 200 Milliliter kaltem Wasser nach und trocknete im Vakuum bei 60°C. Man erhielt 209 Gramm 4-Methyl-5-formylimidazol vom Schmelzpunkt 152,5 bis 156°C, entsprechend einer Ausbeute von 75,3 % der Theorie.

Beispiel 2

162,5 Gramm 2,4-Dimethyl-5-hydroxymethylimidazolhydrochlorid wurden in eine Mischung aus 320 Gramm Salpetersäure (65 Gew.%) und 160 Gramm Wasser eingetragen, 2 Stunden auf 50°C und weitere 4 Stunden auf 80°C erwärmt. Man kühlte das Gemisch auf 20°C ab und stellte das erhaltene Reaktionsgemisch durch Zusatz von 68 Gramm Natronlauge (50 Gew.%) auf pH 8,5. Die Lösung wurde dreimal mit je 250 Gramm n-Butanol extrahiert, die Butanollösung mit Natriumsulfat getrocknet, filtriert und unter Vakuum eingedampft. Der Rückstand wurde in Aceton gelöst und durch Einleiten von Chlorwasserstoffgas das Hydrochlorid des 2,4-Dimethyl-5-formylimidazols ausgefällt. Das rohe Hydrochlorid wurde in Wasser gelöst, mit Natronlauge auf pH 7,5 gestellt und die freie Base mit Chloroform extrahiert. Der Eindampfrückstand wurde zweimal aus 2-Propanol umkristallisiert. Man erhielt 82 Gramm 2,4-Dimethyl-5-formylimidazol (66,1 % der Theorie) vom Schmelzpunkt 149,5 bis 152 °C.

Beispiel 3

50 Gramm 2-Ethyl-4-methyl-5-hydroxymethylimidazolhydrochlorid wurden in einer Mischung von 90 Gramm Salpetersäure (65 Gew.%) und 45 Gramm Wasser gelöst, 2 Stunden bei 40°C und weitere 4 Stunden bei 80°C gerührt, wobei nitrose Gase entweichen. Die Lösung wurde abgekühlt und bei 20°C mit Natronlauge

(50 Gew.%) auf pH 8,5 gestellt. Man extrahierte dreimal mit je 250 Milliliter n-Butanol, trocknete die vereinigten Butanolextrakte über Natriumsulfat, filtrierte und dampfte das Butanol unter Vakuum ein. Der Rückstand wurde mit Aceton gelöst und durch Einleiten von Chlorwasserstoffgas das Hydrochlorid des 2-Ethyl-4-methyl-5-formylimidazols gefällt. Das rohe Hydrochlorid wurde isoliert und wie in Beispiel 2 beschrieben in die freie Base übergeführt. Man erhielt 28,3 Gramm 2-Ethyl-4-methyl-5-formylimidazol vom Schmelzpunkt 79,5 bis 81,0°C (aus Aceton), entsprechend einer Ausbeute von 72,3 % der Theorie.

Beispiel 4

Eine Lösung von 11 Gramm 4-Methyl-5-formylimidazol in 50 Gramm Wasser von 20 - 25°C wurde mit einer Lösung von 16 Gramm 3,4-Dichloranilin in 60 Gramm Wasser und 2 Gramm Salzsäure (36 Gew.%) 15 Minuten am Rückfluß gekocht. Man kühlte auf 20°C ab, saugte die ausgeschiedenen Kristalle ab und kristallisierte sie zweimal aus Wasser um. Man erhielt 19 Gramm 4-Methylimidazolyl-5-(3,4-dichlorphenyl-azomethin) vom Schmelzpunkt 180 bis 182°C, entsprechend einer Ausbeute von 74,8 % der Theorie.

Beispiel 5

55 Gramm 4-Methyl-5-formylimidazol wurden mit 34,7 Gramm Hydroxylammoniumchlorid in 1 000 Gramm Ethanol 30 Minuten am Rückfluß erhitzt (78 - 80°C). Die Lösung wurde unter Vakuum eingedampft und der Rückstand aus 2-Propanol umkristallisiert. Man erhielt 68 Gramm 4-Methylimidazolyl-5-formaldehydoxim-hydrochlorid mit einem Schmelzpunkt von 203 bis 205,5°C, entsprechend einer Ausbeute von 84,2 % der Theorie.

Beispiel 6

55 Gramm 4-Methyl-5-formylimidazol wurden mit 25 Gramm Hydrazinhydrat in 1 000 Gramm Ethanol 30 Minuten am Rückfluß erhitzt (78 - 80°C). Man dampfte unter Vakuum ein und kristallisierte den Rückstand zweimal aus 2-Propanol um. Man erhielt 54 Gramm 4-Methylimidazolyl-5-formaldehydhydrazon (= 87,1 % der Theorie) vom Schmelzpunkt 115,2 bis 119,5°C.

Beispiel 7

55 Gramm 4-Methyl-5-formylimidazol wurden mit 55,8 Gramm Semicarbazid-hydrochlorid in einer Mischung aus 1 000 Gramm Ethanol und 200 Gramm Wasser 30 Minuten am Rückfluß erhitzt (80 - 85°C). Man kühlte das Gemisch im Eisbad, saugte den entstandenen Niederschlag ab und trug ihn in 300 Gramm 15-gewichtsprozentiges Ammoniakwasser ein. Man rührte 10 Minuten, saugte den Niederschlag ab und kristallisierte aus Methanol um. Man erhielt 76 Gramm 4-Methylimidazolyl-5-formaldehydsemicarbazon vom Schmelzpunkt 233°C (unter Zersetzung), entsprechend einer Ausbeute von 90,9 % der Theorie.

Beispiel 8

11 Gramm 4-Methyl-5-formylimidazol wurden mit 9,1 Gramm Thiosemicarbazid in einer Mischung aus 500 Gramm Ethanol und 100 Gramm Wasser 30 Minuten am Rückfluß (80 - 85°C) erhitzt und unter Vakuum eingedampft. Der Rückstand wurde zweimal aus Wasser umkristallisiert. Man erhielt 16 Gramm (87,3 % der Theorie) 4-Methylimidazolyl-5-formaldehyd-thiosemicarbazon vom Schmelzpunkt 209,6 bis 209,8°C.

Beispiel 9

59,6 Gramm Triphenylphosphin-carbmethoxymethylen wurden in 600 Gramm Ethanol mit 22,4 Gramm 4-Methyl-5-formylimidazol 3 Stunden am Rückfluß erhitzt (80°C). Das Reaktionsgemisch wurde abgekühlt und unter Eiskühlung wurde bei 10 bis 20°C Chlorwasserstoffgas, bis das Gemisch ein pH von 1 aufwies, eingeleitet. Die ausgeschiedenen Kristalle wurden abgesaugt. Man erhielt 24 Gramm 4-Methylimidazolyl-5-acrylsäuremethylesterhydrochlorid (entsprechend einer Ausbeute von 59,3 % der Theorie) mit einem Fp (Methanol) 219 bis 219,2°C.

Beispiel 10

224 Gramm P-Diethoxy-phosphonoessigsäureethylester wurden in 1 000 Gramm Ethanol (wasserfrei) mit 110 Gramm 4-Methyl-5-formylimidazol gerührt und zu der Mischung 180 Gramm einer 30-gewichtsprozentigen Natriummethylatlösung in Methanol langsam zugegeben. Die Mischung wurde 6 Stunden bei 55°C gerührt, filtriert, in das Filtrat wurde unter Eiskühlung Chlorwasserstoffgas eingeleitet, bis das Gemisch ein pH von 1 aufwies. Der Niederschlag wurde abgesaugt, mit 1 000 Gramm Methanol auf 65°C erhitzt, vom ungelösten NaCl abfiltriert und das Filtrat unter Vakuum eingedampft. Es verblieben 115 Gramm 4-Methylimidazolyl-5-acrylsäureethylesterhydrochlorid (77,1 % der Theorie) mit einem Fp (Methanol) von 202,5 bis 205°C.

Beispiel 11

238,5 Gramm 4-Methylimidazolyl-5-acrylsäureethylester-hydrochlorid wurden in 550 Gramm Wasser gelöst und mit 550 Gramm wäßriger Salzsäure (36 Gew.%) versetzt. Die Mischung wurde 80 Minuten am Rückfluß erhitzt (100°C), abgekühlt, der Niederschlag wurde abgesaugt und in 400 Gramm Wasser gelöst. Die

Lösung wurde unter Kühlung mit 2n-NaOH auf pH 5,4 gestellt und über Nacht im Eisbad gekühlt. Die abgeschiedenen Kristalle wurden abgesaugt, mit Eiswasser gewaschen und getrocknet. Man erhielt 120 Gramm 4-Methylimidazolyl-5-acrylsäure (83,2 % der Theorie) mit einem Fp von 210 bis 212°C (Zersetzung).

Beispiel 12

430 Gramm Triphenylphosphin-carbdecyloxymethylen wurden in 1 600 Gramm Ethanol mit 110 Gramm 4-Methyl-5-formylimidazol 3 Stunden am Rückfluß erhitzt (78 - 80°C). Man kühlte das Reaktionsgemisch ab, leitete unter Kühlung Chlorwasserstoffgas, bis das Gemisch ein pH von 1 aufwies, ein und saugte das ausgeschiedene Hydrochlorid (140 Gramm alkoholfeucht) ab. Dieses Salz löste man in 500 Gramm Wasser und stellte die Lösung durch Zugabe von Natronlauge auf pH 8,5. Man erhielt 92 Teile 4-Methylimidazolyl-5-acrylsäuredecylester vom Schmelzpunkt 98,5 bis 99°C, entsprechend einer Ausbeute von 31,5 % der Theorie.

Patentansprüche

1. 5-substituierte 4-Methylimidazole der Formel

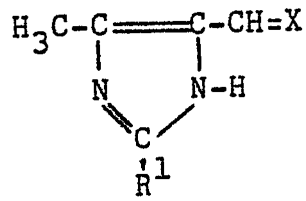 I,

worin X den Rest 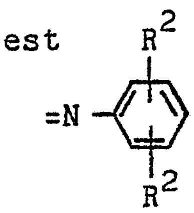, den Rest =NOH, den Rest $=N-NH_2$, den Rest 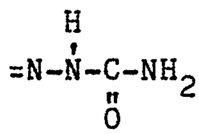, den Rest 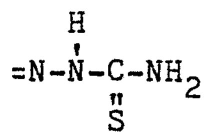 oder den Rest $=CH-COOR^3$ bedeutet, $R^1$, $R^2$ und $R^3$ gleich oder verschieden sein können und jeweils ein Wasserstoffatom oder einen aliphatischen Rest bezeichnen und $R^2$ auch für ein Halogenatom oder eine Alkoxygruppe steht, X auch, wenn $R^1$ einen aliphatischen Rest bedeutet, ein Sauerstoffatom bezeichnen kann.

2. Verfahren zur Herstellung von 5-substituierten 4-Methylimidazolen der Formel

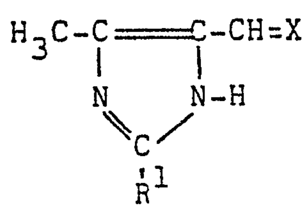 I,

worin X den Rest 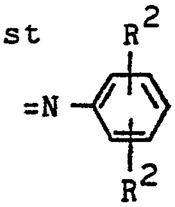, den Rest =NOH, den Rest $=N-NH_2$, den Rest $=N-\overset{H}{N}-\underset{\overset{\parallel}{O}}{C}-NH_2$, den Rest $=N-\overset{H}{N}-\underset{\overset{\parallel}{S}}{C}-NH_2$ oder den Rest $=CH-COOR^3$ bedeutet, $R^1$, $R^2$ und $R^3$ gleich oder verschieden sein können und jeweils ein Wasserstoffatom oder einen aliphatischen Rest bezeichnen und $R^2$ auch für ein Halogenatom oder eine Alkoxygruppe steht, X auch, wenn $R^1$ einen aliphatischen Rest bedeutet, ein Sauerstoffatom bezeichnen kann, dadurch gekennzeichnet, daß man 4-Methyl-5-hydroxymethylimidazole der Formel

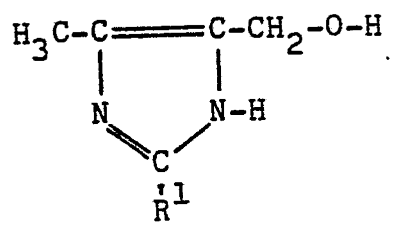

II,

worin $R^1$ die vorgenannte Bedeutung besitzt, mit Oxidationsmitteln zu 4-Methyl-5-formylimidazolen der Formel

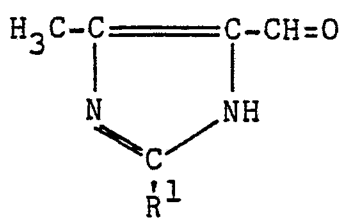

III,

worin $R^1$ die vorgenannte Bedeutung besitzt, oxidiert und gewünschtenfalls die Stoffe III in üblicher Weise mit Stoffen

der Formel

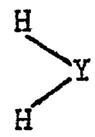
IV,

worin $R^2$ und $R^3$ die vorgenannte Bedeutung besitzen, und Y den Rest 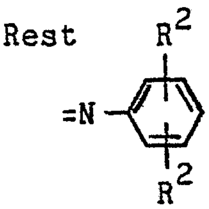, den Rest =NOH, den Rest $=N-NH_2$, den Rest 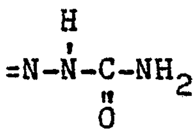 oder den Rest 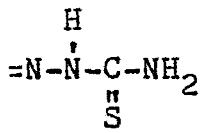 bezeichnet, oder mit Triphenylphosphin-carbalkoxymethylenen oder Dialkoxyphosphonoessigsäurealkylestern umsetzt und gewünschtenfalls die so erhaltenen ß-(4-Methyl)-imidazolyl-(5)-acrylsäurealkylester der Formel

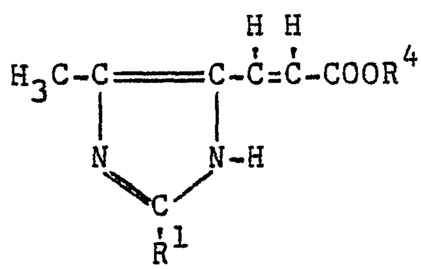
V,

worin $R^1$ die vorgenannte Bedeutung besitzt und $R^4$ einen aliphatischen Rest bezeichnet, in üblicher Weise zu Acrylsäuren der Formel

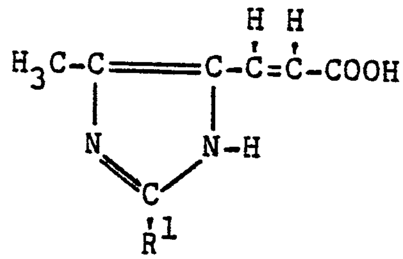

VI,

worin $R^1$ die vorgenannte Bedeutung besitzt, hydrolysiert.